# EUROPEAN PATENT SPECIFICATION

(11) **EP 3 973 927 B1**
(45) Date of publication and mention of the grant of the patent: **31.01.2024**
(21) Application number: 21191379.3
(22) Date of filing: 13.08.2021
(51) Int. Cl.: A61F 2/42

(54) **AN ANKLE PROSTHESIS**
KNÖCHELPROTHESE
PROTHÈSE DE CHEVILLE

(30) Priority: 23.09.2020 GB 202015006
(43) Date of publication of application: 30.03.2022
(73) Proprietor: RLFA Consultants Ltd, 4 Dublin (IE)
(72) Inventor: FLAVIN, Robert, Dublin, 4 (IE)
(74) Representative: Murgitroyd & Company

(56) References cited:
- US-A- 4 755 185
- US-A1- 2006 020 345
- US-A1- 2011 035 019
- US-A1- 2014 081 413

## Description

### Field

The present application relates to ankle prostheses. In particular, the present application relates to durable protheses for ankle arthrodesis and ankle arthroplasty.

### Background

The leg and foot are complex structures with 28 bones and 33 joints that are configured to provide mobility and stability in various surfaces during varying degree of weight-bearing. The foot plays multiple crucial roles in attenuating ground impacts, supporting against gravity, maintaining locomotor stability and transmitting or generating propulsive power during locomotion. The articular cartilage plays a significant role in determining the stress distributions in the joint: the degradation of the cartilage and the underlying bone are the prime reasons of osteoarthritis, causing pain, stiffness and reduction of motion. The most common operative treatments used in end-stage ankle arthritis are ankle arthrodesis and ankle arthroplasty, including Total Ankle Arthroplasty (TAA). TAA is a surgical procedure that replaces the ankle with a prosthesis, thereby providing pain relief while preserving ankle motion. Various prostheses that imitate the anatomical and biomechanical characteristics of the ankle joint are known for use in these procedures, including two and three-component prostheses.

A typical three-component ankle prosthesis comprises a metallic tibial implant, a plastic bearing component, and a metallic talar implant. The talar implant and the tibial implant are configured to be fixed to a patient's tibia and talus respectively. The bearing component is disposed between the tibial implant and the talar implant and is shaped to facilitate sliding on corresponding sliding surfaces of the implants. The sliding surfaces vary between prostheses.

As one example, the Scandinavian total ankle replacement (STAR) prosthesis has a tibial implant with a substantially flat sliding surface and a talar implant with a sliding cylindrical surface with a ridge. A bearing component between the implants is shaped to receive the ridge in a groove. The ridge of the talar implant slides in the groove as the prosthesis is articulated. As another example, the Buechal-Pappas ankle prosthesis has a tibial implant with a substantially flat sliding surface and a talar implant having a sliding surface with a deep, central sulcus. A bearing component between the implants is shaped to match the implants and slide along the sulcus as the prothesis is articulated. The Infinity prosthesis from Wright Medicial Group also has similar sliding surfaces to the Buechal-Pappas ankle prosthesis.

Two-component ankle prostheses differ from three-component ankle prostheses in that the bearing component is fixed to the tibial implant. A review of known ankle prostheses can be found in *"*History and evolution in total ankle arthroplasty" by N.E. Gougoulias et al. in the British Medical Bulletin 2009; 89; page 111.

US2009/198341, Choi discloses a partial bone prosthesis configured for use, for example, on the superior side of the talus or an end of a long bone, such as the tibia. The partial bone prosthesis anchors to the perimeter of the contact patch between the bone and the prosthesis. The prosthesis is attached to the talus without drilling holes through the talus, preserving structural integrity and blood flow within the talus.

US 4755185, Tarr discloses a three-component prosthetic joint comprising a first bone-attachable component having a planar articular surface and a second bone-attachable component having an arcuate articular surface and a substantially congruent bearing component held therebetween and including a system of interlockable flanged grooves which prevent both separation of the arcuate surfaces when interlocked and also medial-lateral movement therebetween.

Many issues remain that need to be addressed with known ankle prostheses. For example, aseptic loosening, subsidence, cyst formation, periprosthetic and fracture of bearing components are all known to occur in known prostheses. An especially challenging issue for many prostheses is the wear and fracture of the bearing component and the resulting incongruency of the articulating surfaces. When combined, these adverse factors result in more frequent side effects and a reduction in the survival probability of a prosthesis.

Hence, there is a need for an improved prosthesis.

### Summary

The invention is defined in claim 1.

Ankle prostheses are described that are suitable for Total Ankle Arthroplasty (TAA) Each described prosthesis has been designed to spread out contact pressure and stress and thereby avoid high values which may damage components of the prosthesis or shorten the lifetime of the prosthesis. Consequentially, the described prostheses have a low rate of wear relative to known prostheses, and the likelihood of adverse effects is decreased.

In particular, the form and location of features of the prostheses have been optimized to spread out contact pressure and stress between implants and the bearing component. In some embodiments, bone coupling features on the implants have also been designed to spread out contact pressure and stress.

A ridge on the bearing component and a groove, for receiving the ridge, on a talar implant cooperate to provide a lateral alignment feature. The lateral alignment feature permits at least limited inversion and eversion of the prosthesis. The lateral alignment feature is also only a small portion of the sliding surfaces, and the remainder of the sliding surfaces is free of features which helps spread out contact pressures and stress.

The sliding surfaces between the bearing component and the tibial implant are flat. These flat sliding surfaces contact each other to form a sliding interface. As this sliding interface is flat, the flat sliding surfaces can remain in contact yet allow the bearing component to slide in any direction, and even twist, relative to the tibial component. This freedom of motion permits increased congruity, i.e., alignment, of the curved sliding surfaces between the talar implant and the bearing component. The freedom of motion also reduces the forces that must be transferred across the prosthesis by the lateral alignment feature. Consequently, the lateral alignment feature can be of a relatively small size. This means the curved sliding surfaces between the talar implant and bearing component are minimally disrupted helping to spread out the contact pressures and stresses.

By analysis of the stresses in the bearing component in the described prostheses, the optimal range of the width and/or radius of the ridge of the lateral alignment feature was found to be 3 to 3.5 mm. Lateral alignment features having such a ridge provide strong lateral alignment yet are significantly smaller than the lateral alignment features in known prostheses.

Revision prostheses are also described which may be used to replace primary (previously implanted) prosthesis. The revision prostheses ensure a strong engagement to the patient's bone whilst still spreading out contact pressure and stress.

### Brief Description of the Drawings

Embodiments of the present invention will now be described, by way of example, with reference to the accompanying drawings, in which:
Figure 1 shows schematically a three-component ankle prosthesis;
Figure 2A shows a talar implant of an ankle prosthesis according to an embodiment of the present invention;
Figure 2B shows a lateral view of the talar implant of figure 2A;
Figure 2C shows an anterior view of the talar implant of figure 2A
Figure 3 shows another talar implant based on figure 2A but with fillets;
Figure 4 shows a bearing component for use with the talar component of figure 2A;
Figure 5A shows a bearing component for use with the talar component of figure 3;
Figure 5B shows a lateral view of the bearing component of figure 5A;
Figure 5C shows an anterior view of the bearing component of figure 5A;
Figure 6A shows a tibial implant of the ankle prosthesis;
Figure 6B shows a lateral view of the tibial implant of figure 6A;
Figure 6C shows an anterior view of the tibial implant of figure 6A;
Figure 7A shows a lateral view of a revision talar implant according to an embodiment of the present invention;
Figure 7B shows an anterior view of the revision talar implant of figure 7A;
Figure 8A shows a lateral view of another revision talar implant for use in a triple arthrodesis procedure;
Figure 8B shows an anterior view of the revision talar implant of figure 8A;
Figure 9A shows a lateral view of revision tibial implants;
Figure 9B shows an anterior view of the revision tibial implants of figure 9A;
Figures 10 shows a finite element model that is used in a process to help assess selected parameters of the present invention;
Figure 11A shows a map of contact stress for a bearing component with a 3 mm wide ridge assessed using the model of figure 10; and
Figure 11B shows a map of contact stress for a bearing component with a 4 mm wide ridge assessed using the model of figure 10.

### Description

An ankle joint can be considered as the mechanical linkage between the talus and the fibula and tibia. As explained above, sometimes this mechanical linkage gets worn or damaged and needs replaced by a prothesis in a Total Ankle Arthroplasty (TAA) procedure.

As mentioned above, TAA prostheses can comprise two-component or three-components.

A two-component prosthesis comprises a tibial implant that fixedly connects to the tibia, and a talar implant that fixedly connects to the talus.

The tibial implant comprises a tibial component which is configured to be affixed to the tibia with a tibial fixing component. The tibial component comprises a strong biocompatible material such as stainless steel, a titanium alloy or a cobalt chrome alloy. There are many known forms of tibial fixing component from shapes that are fixed to the tibial component, which the tibia grows through or around, to surgical cement to adhere the tibial component to the tibia. Fixedly attached to the surface of the tibial component opposite the tibia is a bearing component. The bearing component comprises a plastics material, such as polyethylene. The bearing component has a surface fixedly connected to the tibial component and an opposite outward facing surface directed towards the talus.

The talar implant comprises a talus component with is configured to be affixed to the talus with a talus fixing component. The talus component comprises a strong biocompatible material e.g. stainless steel, a titanium alloy or a cobalt chrome alloy. As with the tibial fixing component, there are many known ways to implement the talus fixing component. The talus component has at least one surface configured to attach to the talus and an outward facing surface directed towards the tibia.

The outward facing surface of the bearing component and the talus component are shaped to correspond with each other. The shaping is such that the corresponding surfaces may slide over one another and thereby permit the tibia to move relative to the talus in a manner mimicking the motion afforded by a natural ankle joint.

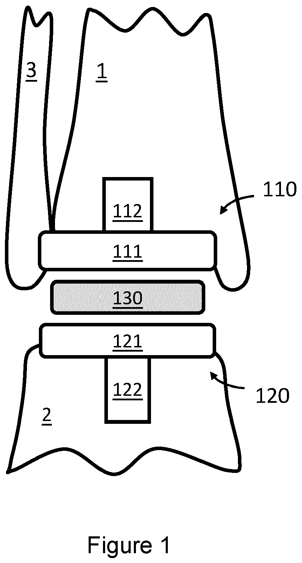

The present invention relates to three-component TAA prostheses. A schematic example of such a three-component TAA prosthesis is shown in figure 1. This is similar to the two-component TAA prosthesis described above. The bearing component 130 is however different because it is not fixed to the tibial component 111. The bearing component 130 is instead free to slide relative to both the tibial component 111 and the talar component 121. An advantage of this three-component prosthesis is that the increased freedom of the bearing component 130 relative to the tibial component 111 permits increased congruity of the sliding interface between the talar implant 120 and the bearing component 130. The increased congruity helps to spread out contact pressure and stress that can cause increased bearing component 130 wear and/or mechanical loosening of the prosthesis.

In some embodiments, the fibula 3 may also be fixed to the tibial component 111 depending on the ankle damage being addressed and the particular prothesis configuration.

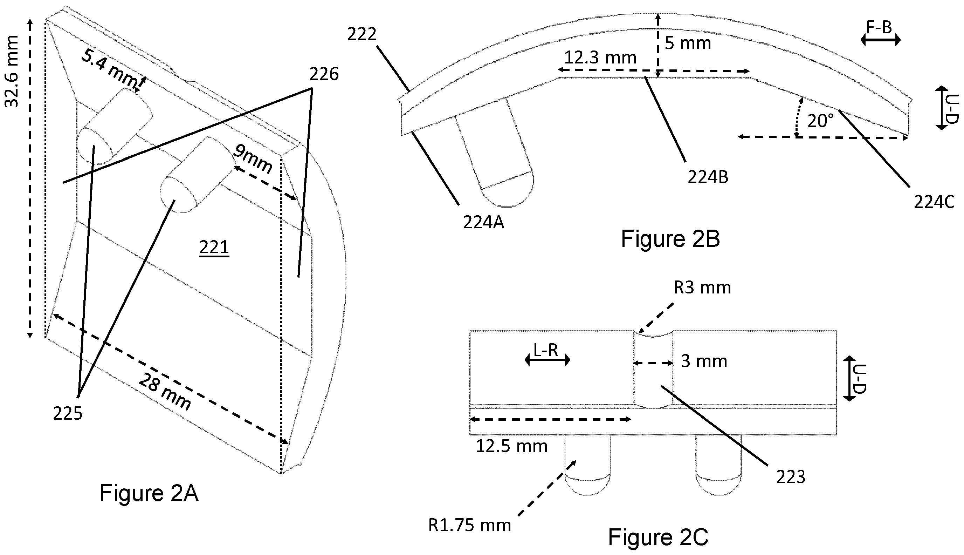

Figures 2A to 2C show an exemplary talar implant 221 for a three-component prosthesis such shown in figure 1B according to an embodiment of the present invention. As with later figures, dimensions are indicated with double headed dashed arrows and are in mm. The front to back (F-B) direction, the lateral (L-R) direction, and vertical directions (U-D) are indicated with the solid double headed arrows. These directions are defined by the parts indicated in the figures as their relative motion and rotation between parts.

The talar implant 221 has the form of a curved cover 28 mm long in the lateral direction and 32 mm long in the front to back direction. The talar implant can be formed from any strong biocompatible implantable material, such as stainless steel, a titanium alloy or a cobalt chrome alloy.

The talar implant 221 has a talus contacting surface which is configured to contact the talus and an outwardly facing surface 222 on the opposite to the talus contacting surface. The outwardly facing surface 222 has cylindrical symmetry and defines a cylindrical surface with a radius of 27 mm. The outwardly facing surface 222 also has a central groove 223 in the middle of the cylindrical surface in the lateral direction. The groove 223 is most preferably defined by a radius of 3 mm and is 3 mm wide (in the lateral direction and runs from the front edge to the back edge of the outwardly facing surface 222. In some similar embodiments, the groove 223 is defined by a radius of between 3 mm and 3.5 mm. In some similar embodiments, the groove 223 has a width between 3 and 3.5 mm wide. As the width and/or defining radius of the groove can range between 3 and 3.5 mm across embodiments, the manufacturing tolerances required to manufacturing the groove are not high. The cylindrical symmetry of the outwardly facing surface 222 further assists in making the talar implant 221 easy to manufacture.

Manufacture is further simplified by the lateral symmetry, i.e., the talar implant 221 has mirror (L-R) symmetry about a central plane as seen in figure 2C.

The talar contacting surface comprises three flat surfaces 224A, 224B, 224C. The three flat surfaces comprise a central flat surface 224B located between two equally sized terminal flat surfaces 224A, 224C. The central flat surface is 12 mm long and joins the two terminal flat surfaces 224A, 224C at respective 70 degrees angle. The two terminal flat surfaces are slanted at a 20 degrees angle from the front to back direction. The thickness of the talar implant 221, which is the distance between the outwardly facing surface 222 and the talar contacting surface is 5 mm at the middle of the central flat surface 224B in the front to back direction. The flat surfaces 224A, 224B, 224C of the talar implant 221 are configured to match with surfaces on a talus that has been surgically prepared e.g. by sawing three matching flats in a talus. Thus, the talar contacting surface ensures the talar implant 221 can be put in strong and stable contact with a surgically prepared talus.

The talar contacting surface further comprises a talar fixing component in the form of two rounded rods 225, that protrude from a terminal flat surface 224A. The rods 225 are 3 mm in diameter and are located in a line so that there is a 5 mm gap between the rods and the front edge of the terminal flat surface and a 9 mm gap to the lateral edges of the terminal flat surface. The talar fixing components are configured to be received in the talus to secure the talar implant 221 to the surgically prepared talus.

In some embodiments, the talar implant 221 may have gutters 226 at the lateral sides that convert the talar implant 221 into a cap for a talus. The positions of gutters are indicated by the dotted lines in figure 2A. Gutters assist with the positioning of the talar implant 221 on the talus. However, embodiments without gutters were found to produce lower stresses in the talar implant 221.

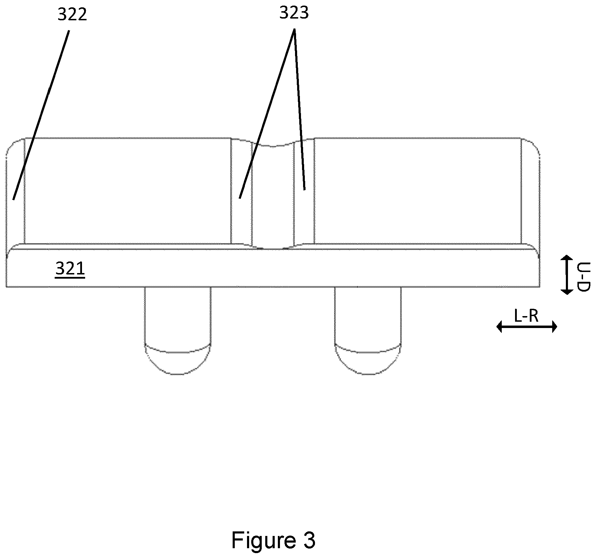

Figure 3 shows an embodiment of a talar implant 321 which is similar to that shown in figures 2A to 2C but with fillets at the edges. The outer edges have a fillet with a 1 mm radius whereas the two edges of the groove 323 have a fillet with a 3 mm radius. Fillets serve to remove sharp edges and thereby improve sliding performance. The fillet of the groove edge affects the ability of the groove to provide lateral alignment, as discussed below. Any processing of an edge that achieves an effect like a fillet is considered equivalent to a fillet e.g. using several chamfers to approximate the shape of a fillet.

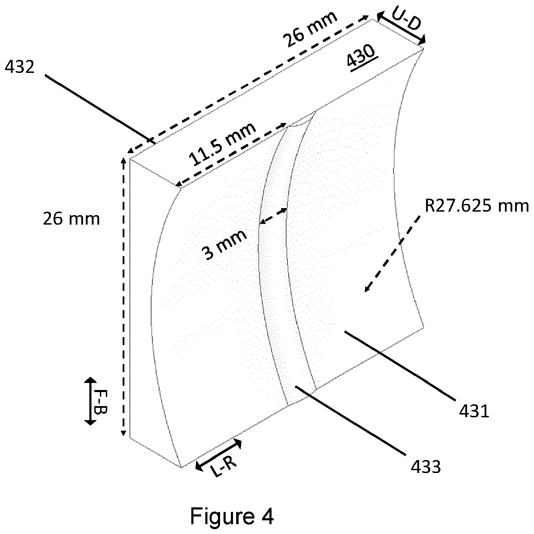

Figure 4 shows a bearing component 430 that is a 26 mm square slab. Other similar embodiments may range in size from 24 to 28 mm, but 26 mm is preferable as it was found to result in the lowest peak stress in the bearing component 430.

The bearing component 430 is designed to match the talar implant 221 shown in figures 2A to 2C. The bearing component 430 is made from a material that slides well over the material of the talar implant 221. In a preferred embodiment, the talar implant 221 comprises a cobalt chrome alloy and the bearing component 430 comprises polyethylene. The bearing component 430 comprises a talar implant contacting surface 431 and a tibial implant contact surface 432 on the opposite surface to the talar implant contacting surface 431. The talar implant contacting surface 431 is configured to contact the outwardly facing surface 222 of the talar implant 221. Therefore, the talar implant contacting surface 431 has cylindrical symmetry and defines a cylindrical surface with radius of 27 mm or more preferably 27.6 mm. The talar implant contacting surface 431 also has a central ridge 433 running in the front to back direction that is shaped to match the groove 223 in the talar implant 221. The cross section of the ridge 433 is defined by a 3 mm radius and the ridge is 3 mm wide in the lateral direction.

The cylindrical symmetry of the talar implant contacting surface 431 and central ridge 433 assists in making the bearing component 430 easy to manufacture. Like the tibial component 221, the bearing component 430 has mirror (L-R) symmetry about a central plane. Manufacture is further simplified by the square-shape and lateral symmetry of the bearing component.

The talar implant 221 and bearing component 430 cooperate to permit sliding of the bearing component 430 over the talar implant 221. Lateral motion is kept to an acceptable degree, to ensure the prosthesis accurately replicates the motion of a natural ankle joint, by engagement of the ridge 433 in the groove 233. The other portions of the contacting surfaces of the talar implant 221 and the bearing component 430 define sliding concentric cylindrical surfaces and thus do not offer any significant restriction of lateral motion or lateral alignment. The groove 223 of the talar implant 221 and the ridge 433 of the bearing component 430 are therefore the only features on the interface between the talar implant 221 and bearing component 430 that maintain lateral alignment of the talar implant 221 and bearing component 430. Maintaining lateral alignment means avoiding lateral motion and avoiding rotations around any axis that is parallel to the vertical direction.

Maintaining lateral alignment is important for the prosthesis to function correctly. However, the restriction of any motion or rotation by a bearing component causes stress and pressure on the bearing component. Over constraining the motion will increase wear on the bearing component 430 and/or will make motion of the prothesis difficult. Conversely, not constraining the motion sufficiently will mean that the prosthesis does not accurately replicate the motion of a natural ankle joint, which is not acceptable and/or may increase wear on the ridge 433 or groove 223.

Consequently, the selection of the form e.g. shape and size of the groove and ridge are important for the functioning of the prosthesis.

The other portions of the contacting surfaces of the talar implant 221 and the bearing component 430, which define sliding concentric cylindrical surfaces, carry a lot of the load placed in the prosthesis. These surfaces are sliding concentric cylindrical surfaces which are large and free of features that may concentrate stress or pressure when load is applied. In some embodiments, the sliding concentric cylindrical surfaces represent over 75% of the sliding surfaces (over 88% for embodiments with a ridge that is 3 mm wide). These large unobstructed concentric cylindrical surfaces help to ensure that the stress and pressures are uniformly distributed over the bearing component 430, which helps improve durability of the bearing component 430. The cylindrical surfaces define an axis of rotation of the prosthesis.

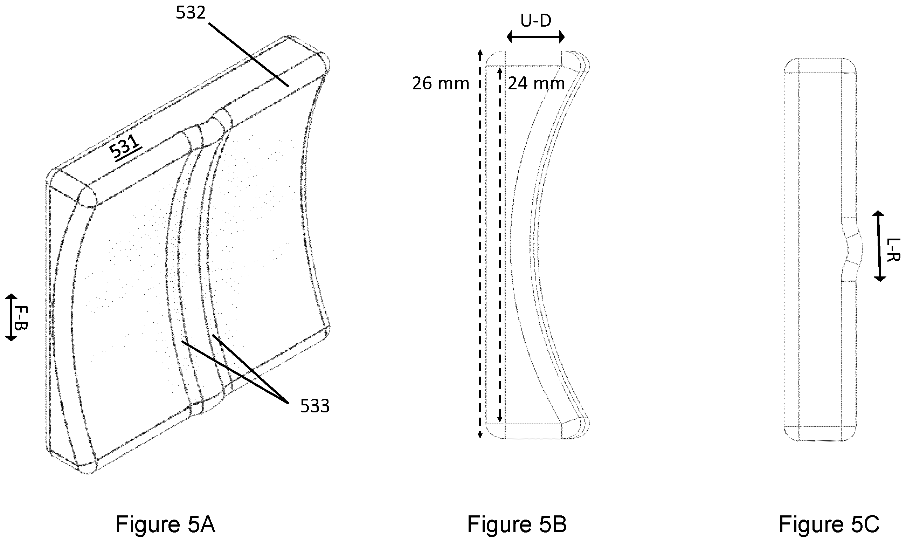

Figures 5A to 5C show another embodiment of the bearing component like that in figure 4 but with filleted edges. This bearing component 531 is suitable for use with the filleted talar implant 321 shown in figure 3. The outer corners of this bearing component 531 have a fillet with a 1 mm radius whereas the two sides 533 of the ridge have a fillet with a 3 mm radius. As before, fillets serve to remove sharp edges and thereby improve sliding performance. The filleted groove and filleted ridge provide a stable lateral restoring force, which is applied over a large area of the bearing component 531. All the lateral aligning forces are applied through the ridge and having a fillet 533 help to avoid stress from the lateral aligning forces concentrating at the foot of the ridge. The use of a fillet also means that if the ridge should temporarily leave the groove it will be easier to reseat the ridge and the ridge will less likely to be damaged e.g. nicked during leaving or reseating process. In this way the prosthesis can deal with at least some inversion and eversion of the prosthesis.

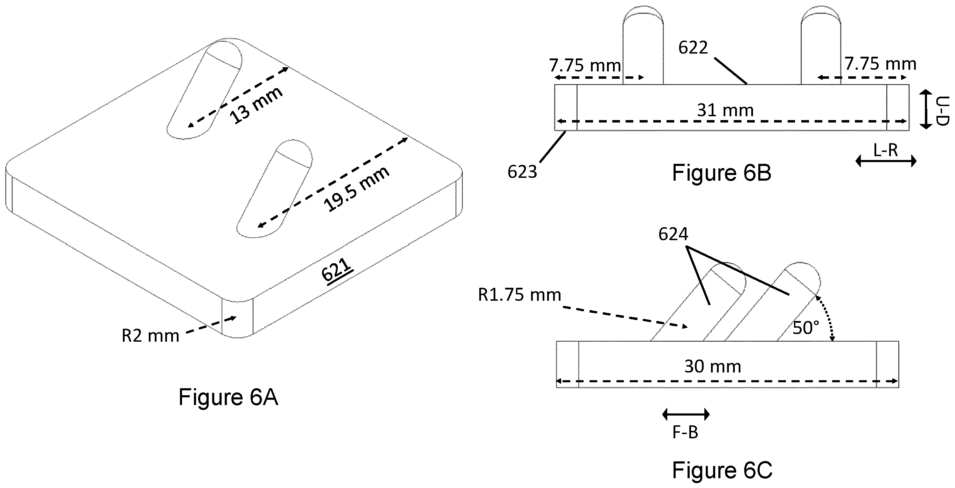

Figures 6A to 6C shows the tibial implant 621 of the three-component prosthesis shown in figure 1B. The tibial implant 621 comprises the same material as the talar implant 221. The tibial implant 621 is a rectangular slab 31 mm long in the lateral direction and 30 mm in the front to back direction, the edges of the rectangle have a 2 mm fillet. The tibial implant 621 has a tibial contacting surface 622 and a flat outwardly facing surface 623.

The tibial contacting surface 622 defines a flat surface that is configured to match with a flat surface on a surgically prepared tibia. In this way, the tibia implant 621 can be put in stable contact with a surgically prepared tibia.

The tibial contacting surface 622 further comprises a tibial fixing component in the form of two rounded rods 624 that protrude from the flat outwardly facing surface 623. The rods are both 3 mm in diameter, parallel, and inclined at an angle of 50° from the flat outwardly facing surface 623. The first rod is disposed on the flat outwardly facing surface 8 mm from the left edge and 13 mm from the back edge. The second rod is disposed on the flat outwardly facing surface 8 mm from the right edge and 20 mm from the back edge. The two rods are configured to be received in a tibia to secure the tibial implant 621 to the tibia. In similar embodiments, the rods are inclined at any angle between 50 and 55°.

The flat outwardly facing surface 623 is configured to slide over the flat tibial facing surface 432 of the bearing component 430. As the outwardly facing surface 623 and tibial facing surface 432 are flat, they provide sliding surfaces that facilitate any relative motion between the tibial implant 621 and the bearing component 421 that does not involve motion in the vertical direction. These flat sliding surfaces thus facilitate rotation around an axis parallel to the vertical direction or translation in the front to back or lateral direction. Therefore, the flat sliding surfaces permit freedoms of motion that increases congruity of the talar implant contacting surface 431 and the outwardly facing surface 222 of the talar implant 221, so helping to reduce pressure and stress in the bearing component. The flat sliding surfaces therefore permit sliding in multiple directions including the lateral direction (L-R), which is aligned with the axis of rotation of the prosthesis.

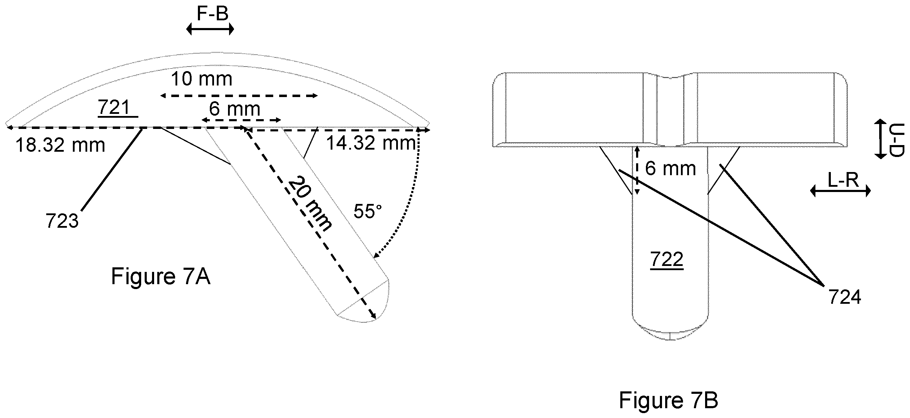

Figures 7A and 7B show a revision talar implant 721 according to another embodiment of the present invention. This revision talar implant 721 differs from those previously described in that it has a single talus fixing component 722 and a flat talus contacting surface 723. The revision talar implant 721 is a revision implant that is suitable for use with patients having an earlier talar implant that has failed or worn. The revision talar implant 721 may also be used with patients having an ankle condition that prevents their talus being surgically prepared in the manner needed to couple to the above described talar implants.

Like the tibial component 221 and the bearing component 430, manufacture of the revision talar implants is simplified by their lateral symmetry, i.e., the revision talar implants have mirror (L-R) symmetry about a central plane, as seen in figure 7B. The single talus fixing component 722 is a 20 mm long rounded rod attached to the flat talus contacting surface 723 at a 55° angle. The interface between the single rod and the flat talus surface 723 extends over 6 mm along the flat talus contacting surface 723. The rod defines an axis that is laterally centred, 18 mm from the front, and 14 mm from the back at the point the axis crosses the flat talus contacting surface 723. In other embodiments the rod may be located at other positions on the flat talus surface 723.

The single rod is supported by a brace 724 protruding from the flat talus contacting surface 723. The brace extends to 6 mm away from the flat talus contacting surface 723 and extends over 10 mm on the flat talus contacting surface 723. The larger flatter surface of the flat talus contacting surface 723 relative to the talar implant shown in figures 2A to 2C ensures the revision talar implant 721 can be stably mounted on a suitably surgically prepared talus. Relatedly, the single well braced rod enables a strong, central and stable connection to the surgically prepared tibia. The single rod is located at a different location and is a different form to the rods of the talar implants described earlier with reference to figures 2A to 3. Consequently, even if the earlier described talar implant 221 has been previous implanted and then removed, the revision talar implant 721 may still be securely affixed to the talus. In similar embodiments of the revision talar implant, the respective single rod is inclined at any angle between 50 and 55° and has a length between 15 and 25 mm long.

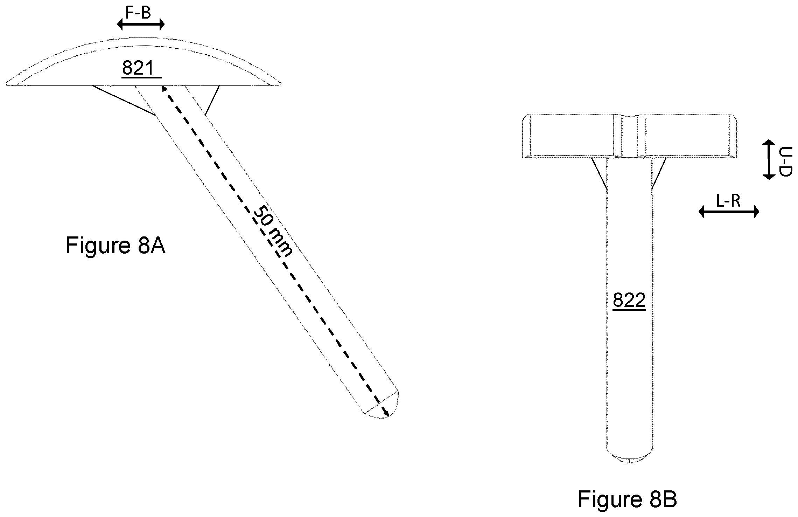

Figures 8A and 8B show a longer revision talar implant 821. The longer revision talar implant 821 is similar the revision talar implant 721 shown in figures 7A and 7B except the single talus fixing component 822 is a single rounded rod that is 50 mm long. The length of this single rod makes the longer revision talar implant 821 suitable for triple arthrodesis i.e. hindfoot fusion procedures. In some embodiments of the longer revision talar implant, the respective single rod is inclined at any angle between 50 and 55° and has a length between 35 and 65 mm long.

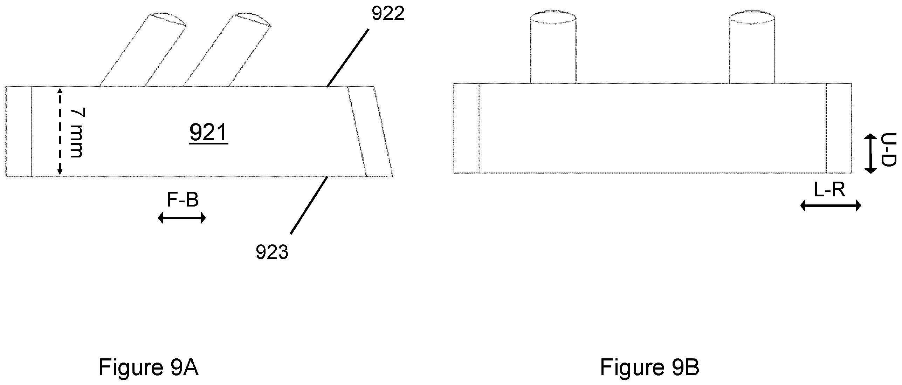

Figure 9A and 9B show embodiments of a revision tibial implant 921. The revision tibial implant 921 is a revision implant that is suitable for use with patients having a primary (earlier implant) tibial implant that has failed or worn. This revision tibial implant 921 may also be used with patients having an ankle condition that prevents their tibia being surgically prepared in the manner needed to couple to the above described tibial implants.

This revision tibial implant 921 differs from those described in relation to figures 6A to 6C in that it is thicker and the back edge 922 is angled. As a result of the slanted back edge 923, the tibial contacting surface 922 is shorter in the front to back direction than the flat outwardly facing surface 923. This means that less tibial surface is required to contact the tibial implant 921 without excessive reduction of the flat outwardly facing surface 923 that contacts the bearing component. The difference between the tibial contacting surface 922 and the flat outwardly facing surface 923 is significant as the distance between these surfaces i.e. the revision tibial implant 921 thickness is 7 mm. In similar embodiments, the tibial implant thickness may be 11 mm or any thickness in the range 5 to 15 mm. As the surface of a damaged tibia is removed, the tibia generally gets narrower, consequently the revision tibial implant 921 permits good attachment to the narrow and shorter tibia without reducing the flat outwardly facing surface 923 that contacts the bearing component.

The selection of the parameters of the prosthesis according to the present invention were assisted using finite element analysis. This method of parameter selection assistance avoids the painful, complicated and invasive nature of in vivo assessment.

The finite element analysis starts by extracting the surface geometry of a subject's ankle from a CT scan. The Young's modulus values for the extracted CT scan values were then determined using conversion software (BoneMat). Next gait data of the subject were obtained using a movement analysis system, with surface markers rigidly secured in the various anatomical landmarks of the tibia and foot. The gait data was normalised in time so that the heel strike occurs at 0% and toe-off occurs at 100% of the stance phase of the gait cycle. The ankle orientation at different time points was also determined.

Next, concentric cylinders were fitted to the modelled talar and tibial contact surfaces and the centre of this concentric cylinder along the axis connecting the malleoli was used for the rotation of foot and tibia in case of dorsiflexion and plantarflexion; and the line perpendicular to the frontal plane of the tibia was used to define inversion and eversion. The articular surfaces of the ankle joint where then resected and replaced with a considered prosthesis. This process is effectively performing a virtual surgical procedure with the considered prosthesis.

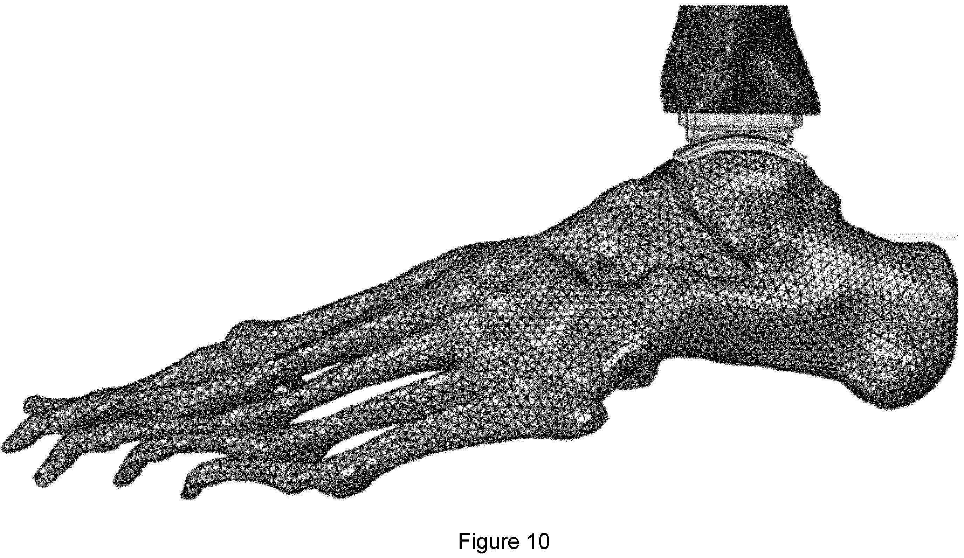

A tetrahedral mesh was then generated for use in finite element software (Abaqus). An example of such a tetrahedral mesh is shown in figure 10. The finite element software was then used to predict maps of von Mises stresses and peak contact pressure for prosthesis with various parameters in various phases of walking gait (heel strike, midstance, heel rise, and Toe-off).

The von Mises stress predicts the yielding of material under complex loading. A material experiencing a stress that its greater than its yield limit may plastically deform resulting in localised wear leading to the failure of the material. Failure of the material may also release large numbers of particles that can incite a chronic inflammatory process which leads to osteolysis, progressing to cause aseptic loosening of the prosthesis. Therefore, the maps of von Mises stress and peak contact pressure, the peak values in these maps, and the distribution of values within these maps be used to assess the performance and durability of prostheses.

Comparison of these maps for various parameters of a prosthesis allows optimization of the various parameters. Examples of the various parameters of the prothesis that were optimized in this way comprise the radius of the articular surface of the talar implant and the corresponding talar contacting surface of the bearing component; the groove radius and width; and the radii of various fillets. The improvement in the resultant optimized prosthesis was also validated against existing TAA prostheses. Due to the nature of stress and loading, these parameters are interconnected for example the stress around the groove is dependent on the radius and width of the groove as well as the radius of the fillets that are applied to the groove.

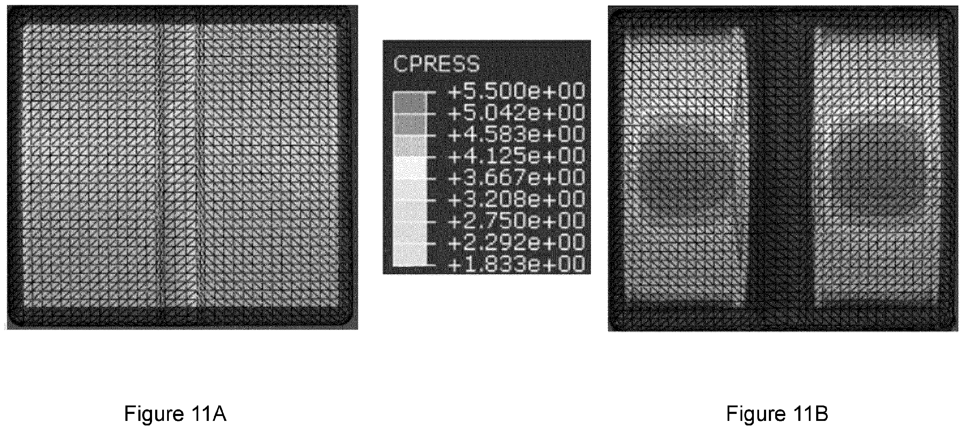

An example of a finite element analysis result is shown in figures 11A and 11B, which show the maps of contact stresses in the midstance phase of gait for two configurations of 3 mm radius ridges: one with a 3 mm wide ridge (figure 11A) and one with a 4 mm width ridge (figure 11B). Clearly, the 3 mm wide ridge results in a more uniform stress map that avoids the two circular areas of high pressure seen on the 4 mm wide ridge. Therefore, from the point of view of reducing or spreading out stress values the 3 mm wide ridge is preferable.

Using this process, the various parameters in the above described prosthesis have been selected to avoid excessive wear, thereby avoiding prosthesis failure and increasing the lifetime of the prosthesis.

The implants and bearing components were therefore developed whilst monitoring the von Mises stress distribution and contact pressure distribution for the three-component (mobile bearing) protheses.

Thus, while a variety of specific primary and revision implants have been described and illustrated above it will be appreciated that the widths and lengths of their surfaces can vary according to the anatomical size of the tibia and talus into which they are implanted, while the shape and radius of the illustrated groove and ridge will remain substantially constant across the ranges of widths and lengths.

## Claims

1. A prosthesis for total ankle arthroplasty, comprising:
a talar implant (221);
a tibial implant (621); and
a bearing component (430) disposed between the tibial implant and talar implant and configured to slide relative to the talar implant to facilitate motion of the prosthesis,
wherein:
the bearing component and the talar implant have corresponding sliding surfaces (222, 431), the corresponding sliding surfaces defining concentric cylindrical surfaces and an axis of rotation, the axis of rotation defining a first direction;
the concentric cylindrical surface of the bearing component (431) have a ridge (433) configured to slide in a corresponding groove (223) in the concentric cylindrical surface (222) of the talar implant;
the alignment of the bearing component and the talar implant, along the axis of rotation, is only due to the interaction of the ridge and the groove; and
the bearing component and the tibial implant have corresponding flat sliding surfaces (623,432); and the bearing component is configured to slide relative to the tibial implant in multiple directions including the first direction, **characterised in that** a cross-section of the ridge defines a radius of between 3 and 3.5 mm.

2. The prosthesis of claim 1, wherein the ridge is between 3 and 3.5 mm wide measured in a direction parallel to the axis of rotation.

3. The prosthesis of any preceding claim, wherein all external corners of the tibial implant, talar implant and bearing component are formed with fillets.

4. The prosthesis of any preceding claim, wherein the bearing component comprises polyethylene, and the tibial implant and talar implant comprise a cobalt chrome alloy.

5. The prosthesis of any preceding claim, wherein the bearing component defines a 26 mm square.

6. The prosthesis of any preceding claim, wherein the talar implant has a talus contacting surface that comprises a flat central surface (224B) between two equally sized terminal flat surfaces (224A, 224C) that are both disposed at the same angle from the flat central surface.

7. The prosthesis of claim 6, wherein two rods (225) perpendicularly project from one of the two terminal flat surfaces (224A) for securing the talar implant to a talus.

8. The prosthesis of any preceding claim, wherein:
the tibial implant (621) has a tibial contacting surface (622) and a parallel and opposing bearing component contacting surface (623); and
two rods (624) project from the tibial contacting surface (224A) at an angle of between 50 and 55 degrees for securing the tibial implant to a tibia.

9. The prosthesis of any preceding claim, wherein:
the talar implant (721) has a talus contacting surface (723) that comprises a flat surface;
a rod (722) projects from the talus contacting surface at an angle of between 50 to 55 degrees for securing the talar implant to a talus; and
one or more supports (724) extending from the talus contacting surface to support the rod.

10. The prosthesis of claim 9, wherein the rod (722) extends for either: 20 mm or 50 mm from the talus contacting surface (723).

11. The prosthesis of any preceding claim, wherein the tibial implant (921) has a tibial contacting surface (922) and a parallel bearing component contacting surface (923) on the opposite side to the tibial contacting surface, and wherein the tibial contacting surface has a smaller area than the bearing component contacting surface.

12. The prosthesis of claim 11, wherein the tibial contacting surface and the bearing component contacting surface are separated by between 7 and 11 mm.

13. The prosthesis of any preceding claim, wherein the concentric cylindrical surfaces are defined by a radius of either: 27, 27.6, or 27.625 mm.

## Patentansprüche

1. Eine Prothese für eine totale Sprunggelenk-Endoprothese, die Folgendes beinhaltet:
ein Talusimplantat (221);
ein Tibiaimplantat (621); und
eine lasttragende Komponente (430), die zwischen dem Tibiaimplantat und
Talusimplantat angeordnet ist und konfiguriert ist, um relativ zu dem Talusimplantat zu rutschen, um die Bewegung der Prothese zu erleichtern,
wobei:
die lasttragende Komponente und das Talusimplantat korrespondierende Rutschflächen (222, 431) aufweisen, wobei die korrespondierenden Rutschflächen konzentrische zylindrische Flächen und eine Rotationsachse definieren, wobei die Rotationsachse eine erste Richtung definiert;
die konzentrische zylindrische Fläche der lasttragenden Komponente (431) eine Rippe (433) aufweist, die konfiguriert ist, um in einer korrespondierenden Rille (223) in der konzentrischen zylindrischen Fläche (222) des Talusimplantats zu rutschen;
die Ausrichtung der lasttragenden Komponente und des Talusimplantats, entlang der Rotationsachse, nur wegen der Interaktion der Rippe und der Rille besteht; und
die lasttragende Komponente und das Tibiaimplantat korrespondierende flache
Rutschflächen (623, 432) aufweisen; und die lasttragende Komponente konfiguriert ist, um relativ zu dem Tibiaimplantat in mehrere Richtungen, umfassend die erste Richtung, zu rutschen, **dadurch gekennzeichnet, dass**
ein Querschnitt der Rippe einen Radius zwischen 3 und 3,5 mm definiert.

2. Prothese gemäß Anspruch 1, wobei die Rippe gemessen in einer Richtung parallel zu der Rotationsachse zwischen 3 und 3,5 mm breit ist.

3. Prothese gemäß einem der vorhergehenden Ansprüche, wobei alle externen Ecken des Tibiaimplantats, Talusimplantats und der lasttragenden Komponente mit Abrundungen gebildet sind.

4. Prothese gemäß einem der vorhergehenden Ansprüche, wobei die lasttragende Komponente Polyethylen beinhaltet und das Tibiaimplantat und Talusimplantat eine Cobaltchromlegierung beinhalten.

5. Prothese gemäß einem der vorhergehenden Ansprüche, wobei die lasttragende Komponente ein 26-mm-Quadrat definiert.

6. Prothese gemäß einem der vorhergehenden Ansprüche, wobei das Talusimplantat eine Talusberührungsfläche aufweist, die eine flache mittige Fläche (224B) zwischen zwei gleich großen endständigen flachen Flächen (224A, 224C) beinhaltet, die beide im selben Winkel von der flachen mittigen Fläche angeordnet sind.

7. Prothese gemäß Anspruch 6, wobei zwei Stäbe (225) senkrecht von einer der zwei endständigen flachen Flächen (224A) zum Sichern des Talusimplantats an einem Talus vorstehen.

8. Prothese gemäß einem der vorhergehenden Ansprüche, wobei:
das Tibiaimplantat (621) eine Tibiaberührungsfläche (622) aufweist und eine parallele und gegenüberliegende Berührungsfläche (623) für die lasttragende Komponente aufweist; und
zwei Stäbe (624) von der Tibiaberührungsfläche (224A) in einem Winkel zwischen 50 und 55 Grad zum Sichern des Tibiaimplantats an einer Tibia vorstehen.

9. Prothese gemäß einem der vorhergehenden Ansprüche, wobei:
das Talusimplantat (721) eine Talusberührungsfläche (723) aufweist, die eine flache Fläche beinhaltet;
ein Stab (722) von der Talusberührungsfläche in einem Winkel zwischen 50 und 55 Grad zum Sichern des Talusimplantats an einem Talus vorsteht; und
sich eine oder mehrere Stützen (724) von der Talusberührungsfläche erstrecken, um den Stab zu stützen.

10. Prothese gemäß Anspruch 9, wobei sich der Stab (722) entweder 20 mm oder 50 mm von der Talusberührungsfläche (723) erstreckt.

11. Prothese gemäß einem der vorhergehenden Ansprüche, wobei das Tibiaimplantat (921) eine Tibiaberührungsfläche (922) und eine parallele Berührungsfläche (923) für die lasttragende Komponente auf der gegenüberliegenden Seite der Tibiaberührungsfläche aufweist und wobei die Tibiaberührungsfläche einen kleineren Bereich als die Berührungsfläche für die lasttragende Komponente aufweist.

12. Prothese gemäß Anspruch 11, wobei die Tibiaberührungsfläche und die Berührungsfläche für die lasttragende Komponente um zwischen 7 und 11 mm getrennt sind.

13. Prothese gemäß einem der vorhergehenden Ansprüche, wobei die konzentrischen zylindrischen Flächen durch einen Radius von entweder 27, 27,6 oder 27,625 mm definiert sind.

## Revendications

1. Une prothèse pour arthroplastie totale de la cheville, comprenant :
un implant talien (221) ;
un implant tibial (621) ; et
un composant d'appui (430) disposé entre l'implant tibial et l'implant talien et configuré pour coulisser par rapport à l'implant talien afin de faciliter le mouvement de la prothèse,
dans laquelle :
le composant d'appui et l'implant talien ont des surfaces coulissantes correspondantes (222, 431), les surfaces coulissantes correspondantes définissant des surfaces cylindriques concentriques et un axe de rotation, l'axe de rotation définissant une première direction ;
la surface cylindrique concentrique du composant d'appui (431) a une nervure (433) configurée pour coulisser dans une rainure correspondante (223) dans la surface cylindrique concentrique (222) de l'implant talien ;
l'alignement du composant d'appui et de l'implant talien, le long de l'axe de rotation, est uniquement dû à l'interaction de la nervure et de la rainure ; et
le composant d'appui et l'implant tibial ont des surfaces coulissantes plates correspondantes (623, 432) ; et le composant d'appui est configuré pour coulisser par rapport à l'implant tibial dans de multiples directions y compris la première direction, **caractérisée en ce que**
une coupe transversale de la nervure définit un rayon compris entre 3 et 3,5 mm.

2. La prothèse de la revendication 1, dans laquelle la nervure a une largeur comprise entre 3 et 3,5 mm mesurée dans une direction parallèle à l'axe de rotation.

3. La prothèse de n'importe quelle revendication précédente, dans laquelle tous les coins externes de l'implant tibial, de l'implant talien et du composant d'appui sont formés avec des arrondis.

4. La prothèse de n'importe quelle revendication précédente, dans laquelle le composant d'appui comprend du polyéthylène, et l'implant tibial et l'implant talien comprennent un alliage cobalt-chrome.

5. La prothèse de n'importe quelle revendication précédente, dans laquelle le composant d'appui définit un carré de 26 mm.

6. La prothèse de n'importe quelle revendication précédente, dans laquelle l'implant talien a une surface de contact avec le talus qui comprend une surface centrale plate (224B) entre deux surfaces plates terminales de dimensions égales (224A, 224C) qui sont toutes les deux disposées selon le même angle comparé la surface centrale plate.

7. La prothèse de la revendication 6, dans laquelle deux tiges (225) font perpendiculairement saillie à partir de l'une des deux surfaces plates terminales (224A) pour fixer l'implant talien à un talus.

8. La prothèse de n'importe quelle revendication précédente, dans laquelle :
l'implant tibial (621) a une surface de contact tibial (622) et une surface de contact avec le composant d'appui parallèle et opposée (623) ; et
deux tiges (624) font saillie à partir de la surface de contact tibial (224A) selon un angle compris entre 50 et 55 degrés pour fixer l'implant tibial à un tibia.

9. La prothèse de n'importe quelle revendication précédente, dans laquelle :
l'implant talien (721) a une surface de contact avec le talus (723) qui comprend une surface plate ;
une tige (722) fait saillie à partir de la surface de contact avec le talus selon un angle compris entre 50 et 55 degrés pour fixer l'implant talien à un talus ; et
un ou plusieurs supports (724) s'étendant à partir de la surface de contact avec le talus afin de soutenir la tige.

10. La prothèse de la revendication 9, dans laquelle la tige (722) s'étend sur : soit 20 mm, soit 50 mm à partir de la surface de contact avec le talus (723).

11. La prothèse de n'importe quelle revendication précédente, dans laquelle l'implant tibial (921) a une surface de contact tibial (922) et une surface de contact avec le composant d'appui parallèle (923) sur le côté opposé à la surface de contact tibial, et dans laquelle la surface de contact tibial a une aire plus petite que la surface de contact avec le composant d'appui.

12. La prothèse de la revendication 11, dans laquelle la surface de contact tibial et la surface de contact avec le composant d'appui sont séparées d'entre 7 et 11 mm.

13. La prothèse de n'importe quelle revendication précédente, dans laquelle les surfaces cylindriques concentriques sont définies par un rayon de : 27, 27,6, ou 27,625 mm.
